# EUROPEAN PATENT APPLICATION

(11) **EP 4 492 033 A1**
(43) Date of publication of application: **15.01.2025**
(21) Application number: 23185460.5
(22) Date of filing: 14.07.2023

(54) **NON-PURIFIED CSF AND CSF-DERIVED EVS MARKERS FOR PATIENTS WITH ALZHEIMER`S, PARKINSON`S AND LEWY BODY DEMENTIA**

(71) Applicant: VITO NV, 2400 Mol (BE); Universiteit Antwerpen, 2000 Antwerpen (BE)
(72) Inventor: HIRSCHBERG, Yael, 2400 Mol (BE); MERTENS, Inge, 2400 Mol (BE)
(74) Representative: Arnold & Siedsma

(57) **Abstract**

The present invention is directed to the field of dementia, providing non-purified CSF and CSF-derived EVs markers for differential dementia diagnosis in patients with Alzheimer's, Parkinson's and Lewy body dementia. It not only provides the markers, but equally the method in using said markers for differential dementia diagnosis amongst the aforementioned patients.

## Description

### FIELD OF THE INVENTION

The present invention is directed to the field of dementia, providing non-purified CSF and CSF-derived EVs markers for differential dementia diagnosis in patients with Alzheimer's, Parkinson's and Lewy body dementia. It not only provides the markers, but equally the method in using said markers for differential dementia diagnosis amongst the aforementioned patients.

### BACKGROUND TO THE INVENTION

Dementia is a leading cause of death worldwide, with increasing prevalence as global life expectancy increases . The most common neurodegenerative disorder is Alzheimer's disease (AD), followed by diffuse Lewy body disease (DLBD), comprising of dementia with Lewy bodies (DLB) and Parkinson's disease dementia (PDD). Whereas AD is primarily characterized by cognitive dysfunctioning and neuropsychiatric symptoms, DLB and PDD are additionally featured by the movement disorder. Dementia also affects up to 80% of patients with Parkinson's disease (PD), which is categorised under the umbrella term of PDD, with the first cognitive deficits commonly present the earliest at the time of PD diagnosis (Litvan et al., 2012). In clinical practice, DLBD is frequently misdiagnosed as AD because of the similar clinical and pathological features. To differentiate between PDD and DLB, there is a current debate about whether PDD and DLB are one or different pathologies, but, generally, the one-year rule is applied (McKeith et al., 2017). This rule is solely based on the onset of the cognitive impairment and states that the patient is diagnosed with PDD if dementia develops one year or more after the motor symptoms. In case these two symptoms develop within the same year, or, if the cognitive impairment is observed before the motor symptoms, DLB should be diagnosed (McKeith et al., 2017). This rule is hard to apply in clinical practice, and even more so at the end stage of both diseases due to almost identical characteristics. Studies attempting to differentiate between both DLBDs, have observed that small differences in motor symptoms, typical for PD, such as tremor, rigidity, and akinesia, tend Parkinsonism of increasing severity is observed in DLB patients over time, with a prevalence up to 85%. However, it is often less response to dopaminergic treatments compared to PDD (McKeith et al., 2017). Also cognitive differences have been discussed previously (Smirnov et al., 2020). DLB, particularly in patients with AD, is associated with more severe cognitive deficits, more frequent hallucinations, and a more rapid disease progression (Devanand et al., 2022). It is important to note that medications are typically developed for a single pathology and may have adverse effects when administered to populations with a different pathology. Furthermore, these therapies are often not tested properly in other populations, thus their effects cannot always be accurately predicted. For example, cholinesterase inhibitors (ChEls) are one of the most used medications in dementia. Both neuropathological and imaging studies have shown significant cholinergic deficits in patients with AD and PDD. These deficits may even be more severe in PDD patients than in AD patients (Müller and Bohnen, 2013). While ChEls have been extensively studied for the treatment of cognitive impairment in AD, there have been relatively few clinical trials investigating ChEl treatment in PD. Although, systematic reviews and meta-analyses suggest mainly positive effects of ChEls in PDD, there is also a growing body of research on the effects of ChEls on the rate of falls in PD, which is a major source of disability in the disease, and whose risk can be further increased in the presence of PDD(Pasquini et al., 2021). Research on ChEl treatments for cognitive deficits in non-demented PD patients with cognitive impairment is expanding but has yielded mixed results thus far. Another therapy commonly used in AD is memantine, an NMDA receptor antagonist that modulates glutamatergic neuronal transmission. The benefits of memantine have also been explored in a few randomised controlled trials evaluating patients with PDD and DLB. However, the results regarding its efficacy have been conflicting, and there is insufficient evidence for its use in this population (Kawashima, 2022). Previous research has reported that up to 89% of PDD patients experience at least one neuropsychiatric symptom (Aarsland et al., 2007). However, unlike in AD, the use of atypical antipsychotics in PDD patients poses a clinical challenge due to the blocking of dopamine (D2) receptors by these medications, which can exacerbate motor symptoms in PD (Iketani et al., 2017).
In line with previously mentioned shortcomings, it is most optimal to provide a disease-specific clinical diagnosis already in the prodromal phase. In order to evaluate early cognitive decline, the term MCI (mild cognitive impairment) is often used. MCI can be considered as an intermediate stage between normal cognition and dementia

With this study, we compared the non-purified CSF and CSF-derived EV proteome of AD, PD, PD-MCI, PDD and DLB patients analyzed by label-free mass spectrometry. This provides new biomarkers to further differentiate between the included dementias and may offer new avenues for research into more disease-specific pharmacological therapeutics.

### SUMMARY OF THE INVENTION

In essence the outcome of this study is directed to a method of differentiating cognitive impairment patients with Alzheimers's (AD), Parkinson's Disease (PD), and Lewy Body Dementia (DLB), from one another with even within Parkinson's Disease a differentiation between Parkinson's Disease Mild Cognitive Impairment (PDMCI) and Parkinson's Disease Dementia (PDD), from one another.

The method can be based either on the protein markers identified in non-purified CSF-samples or on the protein markers identified in CSF derived EV's.

For the non-purified CSF samples a total of 39 protein markers have been identified that enable the aforementioned differentiation between cognitive impaired patients. For the CSF derived EV samples a total of 37 protein markers have been identified that enable the differentiation between cognitive impaired patients.

A method of differentiating cognitive impairment patients, and then in particular neurodegeneration patients with Alzheimers's Disease (AD), Parkinson's Disease (PD), and Lewy Body Dementia (DLB) from one another, said method comprising determining within a CSF-derived EV protein sample or within a non-purified CSF protein sample of said patient the presence of;
- at least one protein from the group consisting of IGKV3D-15, CA3, SORCS3, MYH9, TUBB2B, GALNT1, CALML5, SMOC1, SPINT2, BAMBI, CFHR2, EDF1, PLA2G4E, and ARGLU1;
- at least one protein from the group consisting of PLXDC2, SHISA5, CPLANE1, PCDH9, C1RL, SERPINB4, ACTC1, MGP, CD81, IGHV3-30-5, PRKCSH, SLPI, F2R, IGHV2-70D, SPOCK1, SAMD9, SIRPA, PREX1, LDHA, TCOF1, EPHB1, RB1CC1, KRT40, and KRT38; and
- at least one protein from the group consisting of EPHB6, KRT8, EPB42, CENPE, RALB, POR, SMPD3, MAPT, DDX6, ILK, OGN, CTSS, PPP1CC, FLII, IGKV1-8, SHMT1, and RPL35A.

In an enhanced embodiment of the invention, the method further enables the identification of Parkinson's Disease Mild Cognitive Impairment (PDMCI) and Parkinson's Disease Dementia (PDD) amongst patients with cognitive impairment, said method further comprising determining within a CSF-derived EV protein sample or within a non-purified CSF protein sample of said patient the presence of;
- at least one protein from the group consisting of SNCA, MASP1, PLD4, PTPRS, CADM2, EHD2, ACTBL2, and SLIRP;
- at least one protein from the group consisting of HEG1, MOG, PNP, XIRP2, HP, IGG1, SORL1, and TF; and
- at least one protein from the group consisting of WFIKKN2, PTPRN2, TGM5, IGLC1, and ALB.

In an embodiment the method of differentiating cognitive impairment patients with Alzheimer's Disease (AD), Parkinson's Disease (PD), and Lewy Body Dementia (DLB) from one another is only based on the use of non-purified CSF protein samples, in said embodiment the method comprises determining within a non-purified CSF protein sample of said patient the presence of;
- at least one protein from the group consisting of IGKV3D-15, CA3, SORCS3, MYH9, TUBB2B, GALNT1, CALML5, SMOC1, SPINT2 and BAMBI;
- at least one protein from the group consisting of PLXDC2, SHISA5, CPLANE1, PCDH9, C1RL, SERPINB4, ACTC1, MGP, CD81, IGHV3-30-5, PRKCSH, SLPI, F2R, IGHV2-70D and SPOCK1; and
- at least one protein from the group consisting of EPHB6, KRT8, EPB42 and CENPE.

In a further aspect of this embodiment wherein the method is only based on non-purified CSF protein samples, the further identification of Parkinson's Disease Mild Cognitive Impairment (PDMCI) and Parkinson's Disease Dementia (PDD) comprises determining within a non-purified CSF protein sample of said patient the presence of;
at least one protein from the group consisting of SNCA, MASP1, PLD4, PTPRS and CADM2;
- at least one protein from the group consisting of HEG1, MOG and PNP; and
- at least one protein from the group consisting of WFIKKN2 and PTPRN2.

In an embodiment the method of differentiating cognitive impairment patients with Alzheimer's Disease (AD), Parkinson's Disease (PD), and Lewy Body Dementia (DLB) from one another is only based on the use of CSF-derived EV protein samples, in said embodiment the method comprises determining within a CSF-derived EV protein sample of said patient the presence of;
- at least one protein from the group consisting of CFHR2, EDF1, PLA2G4E, and ARGLU 1;
- at least one protein from the group consisting of SAMD9, SIRPA, PREX1, LDHA, TCOF1, EPHB1, RB1CC1, KRT40, and KRT38; and
- at least one protein from the group consisting of RALB, POR, SMPD3, MAPT, DDX6, ILK, OGN, CTSS, PPP1CC, FLII, IGKV1-8, SHMT1, and RPL35A.

In a further aspect of this embodiment wherein the method is only based on CSF-derived EV protein samples, the further identification of Parkinson's Disease Mild Cognitive Impairment (PDMCI) and Parkinson's Disease Dementia (PDD) comprises determining within a CSF-derived EV protein sample of said patient the presence of;
- at least one protein from the group consisting of EHD2, ACTBL2, and SLIRP;
- at least one protein from the group consisting of XIRP2, HP, IGG1, SORL1, and TF; and
- at least one protein from the group consisting of TGM5, IGLC1, and ALB.

The methods as herein provided wherein the identification of cognitive impairment patients with Alzheimers's Disease (AD) is based on determining within a CSF-derived EV protein sample or within a non-purified CSF protein sample of said patient the presence of at least one protein from the group consisting of IGKV3D-15, CA3, SORCS3, MYH9, TUBB2B, GALNT1, CALML5, SMOC1, SPINT2, BAMBI, CFHR2, EDF1, PLA2G4E, and ARGLU1. In particular the presence of at least one protein selected from the group consisting of IGKV3D-15, CA3, SORCS3, MYH9, TUBB2B, GALNT1, CALML5, SMOC1, SPINT2, and BAMBI in a non-purified CSF protein sample of said patient and/or at least one protein selected from the group consisting of CFHR2, EDF1, PLA2G4E, and ARGLU1 in a CSF-derived EV protein sample of said patient.

The methods as herein provided wherein the identification of cognitive impairment patients with Lewy Body Dementia (DLB) is based on determining within a CSF-derived EV protein sample or within a non-purified CSF protein sample of said patient the presence of at least one protein from the group consisting of PLXDC2, SHISA5, CPLANE1, PCDH9, C1RL, SERPINB4, ACTC1, MGP, CD81, IGHV3-30-5, PRKCSH, SLPI, F2R, IGHV2-70D, SPOCK1, SAMD9, SIRPA, PREX1, LDHA, TCOF1, EPHB1, RB1CC1, KRT40, and KRT38. In particular the presence of at least one protein selected from the group consisting of PLXDC2, SHISA5, CPLANE1, PCDH9, C1RL, SERPINB4, ACTC1, MGP, CD81, IGHV3-30-5, PRKCSH, SLPI, F2R, IGHV2-70D, and SPOCK1 in a non-purified CSF protein sample of said patient and/or at least one protein selected from the group consisting of SAMD9, SIRPA, PREX1, LDHA, TCOF1, EPHB1, RB1CC1, KRT40, and KRT38 in a CSF-derived EV protein sample of said patient.

The methods as herein provided wherein the identification of cognitive impairment patients with Parkinson's Disease (PD) is based on determining within a CSF-derived EV protein sample or within a non-purified CSF protein sample of said patient the presence of at least one protein from the group consisting of EPHB6, KRT8, EPB42, CENPE, RALB, POR, SMPD3, MAPT, DDX6, ILK, OGN, CTSS, PPP1CC, FLII, IGKV1-8, SHMT1, and RPL35A. In particular the presence of at least one protein selected from the group consisting of EPHB6, KRT8, EPB42, and CENPE in a non-purified CSF protein sample of said patient and/or at least one protein selected from the group consisting of RALB, POR, SMPD3, MAPT, DDX6, ILK, OGN, CTSS, PPP1CC, FLII, IGKV1-8, SHMT1, and RPL35A in a CSF-derived EV protein sample of said patient.

The methods as herein provided wherein the identification of cognitive impairment patients with Parkinson's Disease Dementia (PDD) is based on determining within a CSF-derived EV protein sample or within a non-purified CSF protein sample of said patient the presence of at least one protein from the group consisting of SNCA, MASP1, PLD4, PTPRS, CADM2, EHD2, ACTBL2, and SLIRP. In particular the presence of at least one protein selected from the group consisting of SNCA, MASP1, PLD4, PTPRS, and CADM2 in a non-purified CSF protein sample of said patient and/or at least one protein selected from the group consisting of EHD2, ACTBL2, and SLIRP in a CSF-derived EV protein sample of said patient.

In an aspect the present invention enables to differentiate HC vs PDMCI and HC vs PDD, said differentiation comprising determining within a CSF-derived EV protein sample or within a non-purified CSF protein sample of said patient the presence of at least one protein from the group consisting of HEG1, MOG, PNP, XIRP2, HP, IGG1, SORL1, and TF. In particular the presence of at least one protein selected from the group consisting of HEG1, MOG, and PNP in a non-purified CSF protein sample of said patient and/or at least one protein selected from the group consisting of XIRP2, HP, IGG1, SORL1, and TF in a CSF-derived EV protein sample of said patient.

In another aspect the present invention enables to differentiate HC vs PD continuum and HC vs DLB (and eventually also AD vs DLB and/or AD vs PD continuum), said differentiation comprising determining within a CSF-derived EV protein sample or within a non-purified CSF protein sample of said patient the presence of at least one protein from the group consisting of WFIKKN2, PTPRN2, TGM5, IGLC1, and ALB. In particular the presence of at least one protein selected from the group consisting of WFIKKN2, and PTPRN2 in a non-purified CSF protein sample of said patient and/or at least one protein selected from the group consisting of TGM5, IGLC1, and ALB in a CSF-derived EV protein sample of said patient. 'PD continuum' as used herein is based on the generally acknowledged concept that there is a continuum between Parkinson's disease with Mild Cognitive Impairment (PDMCI), Lewy body dementia (DLB), and dementia in Parkinson's disease (PDD), wherein the latter two are currently not always recognized as two different disease indications. The term 'PD continuum' accordingly embraces patients within this so named continuum spectrum between cognitive impaired patients with PDMCI, DLB and PDD.

Whitin the methods as provided the identification of cognitive impairment patients with Alzheimer's Disease (AD) is meant to include a differentiation between HC vs AD and/or AD vs DLB (and eventually AD vs PD continuum). The protein markers mentioned in association with AD are unique to AD and do not occur in association with either DLB, PD, PDMCI nor PDD, nor amongst one another.

Within the methods as provided the identification of cognitive impairment patients with Lewy Body Dementia (DLB) is meant to include a differentiation between HC vs DLB and AD vs DLB. For the plurality of patients including a differentiation between DLB and PD continuum, suggesting that DLB and PDD are two different disease indications with differentiating proteins between them. The protein markers mentioned in association with DLB are unique to DLB and do not occur in association with either AD, PD, PDMCI nor PDD.

Within the methods as provided the identification of cognitive impairment patients with Parkinson's Disease (PD) is meant to include a differentiation between PD vs HC and PD vs PDD. Eventually also between PD vs AD or PD vs DLB. When using non-purified CSF protein samples one protein makes a differentiation between DLB vs PDD and can be used as marker to validate that DLB and PDD are two different diseases. The protein markers mentioned in association with PD are unique to PD and do not occur in association with either AD, DLB, PDMCI nor PDD.

Within the methods as provided the protein markers mentioned in relation to the identification and differentiation of Parkinson's Disease Mild Cognitive Impairment (PDMCI) and Parkinson's Disease Dementia (PDD), includes a possible further differentiation amongst Lewy Body Dementia (DLB) and Parkinson's Disease Dementia (PDD) often seen as the same disease indication. In said methods the markers SNCA, MASP1, PLD4, PTPRS, CADM2, EHD2, ACTBL2, and SLIRP, independent whether assessed in a non-purified CSF protein sample or a CSF derived EV protein sample are unique to PDD and used in the identification of cognitive impairment patients with Parkinson's Disease Dementia (PDD). The identification of cognitive impairment patients with Parkinson's Disease Dementia (PDD) is meant to include a differentiation between HC vs PDD and AD vs PDD or PD vs PDD or DLB vs PDD (accepting again that DLB and PDD are two different diseases). The protein markers mentioned in association with PDD are unique to PDD and do not occur in association with either AD, DLB, PDMCI nor PD.

Within the methods as provided the protein markers mentioned in relation to the identification and differentiation of Parkinson's Disease Mild Cognitive Impairment (PDMCI) and Parkinson's Disease Dementia (PDD), includes a possible further differentiation amongst Lewy Body Dementia (DLB) and Parkinson's Disease Dementia (PDD) often seen as the same disease indication. In said methods the markers HEG1, MOG, PNP, XIRP2, HP, IGG1, SORL1, and TF; are unique to PD continuum when seen as the group consisting of PDMCI and PDD, and allows differentiation of HC vs this group and HC vs PDD.

Within the methods as provided the protein markers mentioned in relation to the identification and differentiation of Parkinson's Disease Mild Cognitive Impairment (PDMCI) and Parkinson's Disease Dementia (PDD), includes the possibility of effectively identifying a pathological overlap amongst Lewy Body Dementia (DLB) and Parkinson's Disease Dementia (PDD) often seen as the same disease indication. In said methods the markers WFIKKN2, PTPRN2, TGM5, IGLC1, and ALB are unique to the PD continuum group when seen as the group consisting of PDMCI-DLB-PDD, and allows differentiation of HC vs PDMCI-PDD and HC vs DLB.

Within the methods as provided, whenever reference is made to 'determining at least one protein the group consisting of'; this includes the determining at least 2, 3, 4, 5, 6, 7, 8, 9, 10, 11, 12, 13, 14, 15, 16, 17, 18, 19, 20, 21, 22, 23, 24 or the total number of proteins present within said group.

### BRIEF DESCRIPTION OF THE DRAWINGS

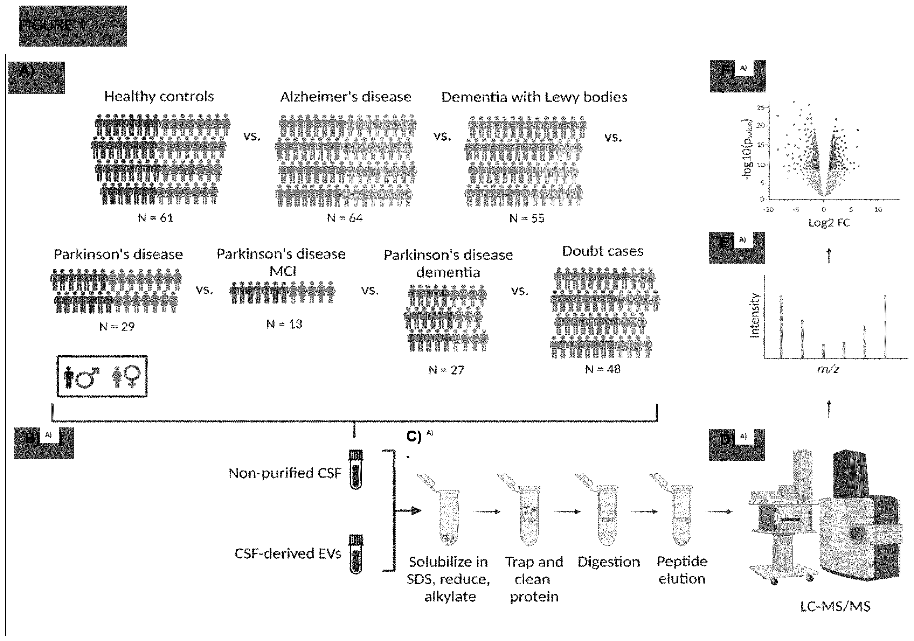

**Figure 1**. Overview of the study. A) illustrates the sample cohort detailed in sections "Study population" and "CSF collection and metadata". B) shows the acquirement of two sample types, as outlined in section "Isolation of extracellular vesicles". C) demonstrates the sample preparation steps for LC-MS/MS, as described in section "Protein extraction and S-trap digestion", D) depicts the instruments used according to section "Liquid chromatography tandem mass spectrometry (LC-MS/MS) analysis", E) demonstrates LC-MS/MS output results being peptide intensity on the Y-axis and m/z on the X-axis, as used in section "Peptide identification". F) is a volcano plot to present the differential data analysis as performed in R, see section "Differential analysis". Abbreviations: CSF, cerebrospinal fluid; EV, extracellular vesicle; FC, fold change; LC-MS/MS, liquid chromatography tandem mass spectrometry; MCI, mild cognitive impairment; m/z, mass to charge ratio. Created with BioRender.com.

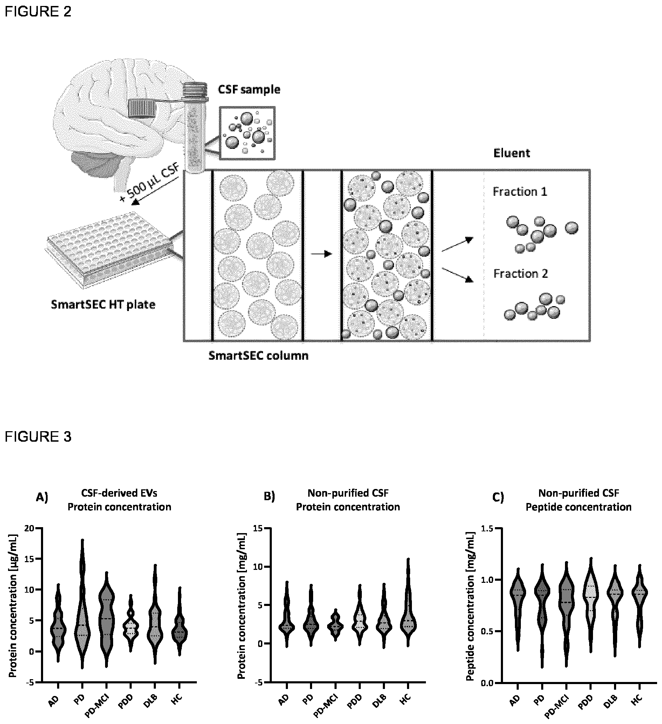

**Figure 2**. Illustration of the SmartSEC EV isolation technique, performed on aliquots number one. Particles that can pass the size exclusion columns are collected in the eluent, while small proteins (up to 400 kDa) enter the bead core and, because of affinity interaction modes, stay trapped. Figure adapted from System Biosciences, with figures from smart.servier.com (licensed under CC BY 3.0).
**Figure 3**. Violin plots of protein and peptide concentration comparisons between different phenotypes, after removing outliers (ROUT, Q=1%). Abbreviations: AD, Alzheimer's disease; PD, Parkinson's disease; PD-MCI, Parkinson's disease with cognitive deficit; PDD, Parkinson's disease dementia; DLB, dementia with Lewy bodies; HC, healthy controls.

### DETAILED DESCRIPTION OF THE INVENTION

### Materials & Methods

### Study population

The study included CSF samples of 249 subjects, including patients with AD, PD, PD-MCI, PDD, DLB and age-matched cognitively healthy controls (HC) (Table 1). Samples were retrospectively selected from the NeuroBiobank of the Institute Born-Bunge (NBB-IBB, federal agency for medicines and health products registration number 190113; Antwerp, Belgium). All participants were originally recruited from the Memory Clinic of the Hospital Network Antwerp (ZNA-Middelheim and ZNA-Hoge Beuken, Antwerp, Belgium) between 1992-2018, as part of their diagnostic clinical work-up.

**Table 1. Demographics and sample information.**

| | **AD** | **PD** | **PD-MCI** | **PDD** | **DLB** | **HC** |
|---|---|---|---|---|---|---|
| **Subjects** - **N** | 64 | 29 | 13 | 27 | 55 | 61 |
| **Age** - **years** | | | | | | |
| Median | 76 | 70 | 80 | 78 | 75 | 65 |
| Minimum - maximum | 56 - 89 | 36 - 83 | 67 - 85 | 66 - 88 | 61 - 90 | 54 - 88 |
| **Storage time** | | | | | | |
| 0 - 5 years | 21 (33%) | 0 (0%) | 0 (0%) | 2 (7%) | 3 (5%) | 11 (18%) |
| 5 - 10 years | 39 (61%) | 1 (3%) | 0 (0%) | 1 (4%) | 3 (5%) | 24 (39%) |
| 10 - 15 years | 4 (6%) | 3 (11%) | 1 (8%) | 3 (11%) | 14 (26%) | 0 (0%) |
| 15 - 20 years | 0 (0%) | 10 (34%) | 2 (15%) | 18 (67%) | 23 (42%) | 26 (43%) |
| >20 years | 0 (0%) | 15 (52%) | 10 (77%) | 3 (11%) | 12 (22%) | 0 (0%) |
| **Sex - N** | | | | | | |
| Male | 32 (50%) | 14 (48%) | 8 (62%) | 16 (59%) | 41 (75%) | 31 (51%) |
| Female | 32 (50%) | 15 (52%) | 5 (38%) | 11 (41%) | 14 (25%) | 30 (49%) |
| **C-fraction** - **N** | | | | | | |
| C1 | 54 (84%) | 11 (38%) | 2 (15%) | 16 (59%) | 33 (60%) | 11 (18%) |
| C2 | 1 (2%) | 0 (0%) | 0 (0%) | 0 (0%) | 0 (0%) | 0 (0%) |
| C3 | 3 (5%) | 0 (0%) | 0 (0%) | 0 (0%) | 0 (0%) | 25 (41%) |
| C4 | 6 (9%) | 0 (0%) | 1 (8%) | 2 (7%) | 5 (9%) | 3 (5%) |
| C5 | 0 (0%) | 0 (0%) | 0 (0%) | 0 (0%) | 2 (4%) | 0 (0%) |
| Unknown | 0 (0%) | 18 (62%) | 10 (77%) | 9 (33%) | 15 (27%) | 22 (36%) |
| **Previous F/T *cycli*** - **N** | | | | | | |
| 0 | 48 (75%) | 24 (83%) | 7 (54%) | 21 (78%) | 35 (64%) | 56 (92%) |
| 1 | 12 (19%) | 4 (14%) | 5 (38%) | 6 (22%) | 15 (27%) | 5 (8%) |
| 2 | 4 (6%) | 0 (0%) | 1 (8%) | 0 (0%) | 2 (4%) | 0 (0%) |
| 3 | 0 (0%) | 1 (3%) | 0 (0%) | 0 (0%) | 3 (5%) | 0 (0%) |
| **Definite diagnosis** - **N** | 14 | 0 | 0 | 2 | 9 | NTA |
| **MMSE score known** - **N** | 48 | 0 | 0 | 0 | 0 | 8 |

| | | | | | | |
|---|---|---|---|---|---|---|
| Abbreviations: AD, Alzheimer's disease; PD, Parkinson's disease; PD-MCl, Parkinson's disease with mild cognitive impairment; PDD, Parkinson's disease dementia; DLB, dementia with Lewy bodies; HC, healthy controls; F/T, freeze/thaw; MMSE, Mini-Mental State Examination; NTA, not applicable; N, number. | | | | | | |

The clinical diagnosis of probable AD was based on the NINCDS/ADRDA criteria of McKhann et al. (McKhann et al., 2011) whereas probable DLB was diagnosed according to the consensus guidelines of McKeith et al. (McKeith et al., 2017). In addition, AD patients had to fulfill the international work group criteria-2 (IWG-2) during clinical diagnosis. Few patients within this cohort also consented to a post-mortem neuropathological immunohistochemical investigation, to either confirm or reject their clinical diagnoses (i.e. resulting into definite AD or DLB) (Table 1: definite diagnosis) (Vermeiren et al., 2015). Subjects were diagnosed with PD according to protocol and criteria as described previously (Engelborghs et al., 2003). PD patients were classified as PD, PD-MCI or PDD based on the conclusion reports of extensive neuropsychological assessments from no more than three months before or after date of sampling. All patients also fulfilled the Diagnostic and Statistical Manual of Mental Disorders-IV-text revision (DMS-IV-TR) criteria (American Psyciatric Association 2010).

Healthy controls were sampled between 2002 and 2017 and had no neurological nor psychiatric antecedents or an organic disease involving the central nervous system. All HC were hospitalised during the time of lumbar puncture and mainly consisted of (i) subjects complaining of low back pain requiring a selective lumbar radiculography; (ii) patients with disorders of the peripheral nervous system (peripheral facial nerve palsy); and (iii) patients with subjective complaints in whom disorders of the central and peripheral nervous system were ruled out by means of an extensive clinical work-up (Engelborghs et al., 2008). Our study was conducted in compliance with the Helsinki Declaration, and Ethics Approval for human sample collection of CSF post-mortem brain obduction was granted by the Medical Ethical Committee of the Middelheim General Hospital (Antwerp, Belgium; approval numbers 2805 and 2806).

### CSF collection and metadata

CSF sampling was performed as described previously (Vermeiren et al., 2013). In short, a lumbar puncture was performed at the L3/L4 or L4/L5 interspace between 08.00 and 10.00 a.m., after overnight fasting and having abstained from smoking for at least 12 hours. Morning medication was administered after the lumbar puncture. A total of 16.5 mL was collected stepwise in five fractions (polypropylene vials (Nalgene; VWR, Leuven, Belgium): fraction C1 (4.5 mL), C2 (1.5 mL), C3 (1.5 mL), C4 (4.5 mL) and C5 (4.5 mL). Fractions C1 to C4 were immediately frozen in liquid nitrogen. Fraction C5 was centrifugated (Centrifuge 5702, rotor A-4-38; Eppendorf, Hamburg, Germany) on 3,000 rpm for 10 minutes, after which the supernatant was divided over three vials, which were then frozen in liquid nitrogen as well.

For this study, the selection of fraction and preceding freeze/thaw (F/T) *cycli* was based upon availability (Table 1). Hemolysis was checked visually to avoid potential oxidation and contamination effects. Six samples of the cohort were observed to be slightly hemolytic. For some AD and HC CSF samples, analyses of Aβ42, total tau (T-Tau), and hyperphosphorylated tau (pTau181) were previously performed by means of ELISA, as part of a routine diagnostic procedure (***Table 1***). Cut-off values were derived from the lower and upper detection limits inherent to the ELISA kits (INNOTEST Aβ(1-42), INNOTEST hTAU-Ag, and INNOTEST pTau181 (Fujirebio, Ghent, Belgium), that is: 125 pg/mL and 2,000 pg/mL for Aβ42, 75 pg/mL and 1,200 pg/mL for T-Tau, and, 15.6 pg/mL and 500 pg/mL for pTau181.

In general, of each frozen CSF sample, two aliquots of 500 µL (aliquot number one and aliquot number two) were taken and re-frozen at -80 °C, inducing an additional F/T cycle. For two exceptions, not sufficient volume of CSF was available for two aliquots and only one aliquot (i.e. aliquots number one) was stored. One other sample had too little volume available for EV isolation, so was only used as aliquot number two. Aliquots number one were used for EV isolation (N = 249), while aliquots number two were not enriched prior to analyses and kept stored at -80 °C until LC-MS/MS (liquid chromatography tandem mass spectrometry) sample preparation (N = 248).

### Isolation of extracellular vesicles

Aliquots number one (500 µL of CSF, N = 249) were thawed on ice and centrifuged 10 minutes at 2,500 x g to remove cell debris prior to EV isolation using the SmartSEC HT kit (System Biosciences) according to manufacturer's instructions **(Error! Reference source not found.).** After preparing the SmartSEC filter plate, samples were loaded into the wells. Following an incubation step of 30 minutes at room temperature, the first SEC fraction was collected by centrifuging the plate for 2 minutes at 500 x g. All centrifugation steps in this study were performed with a centrifuge 5424 R and a fixed angle rotor. To collect the second SEC fraction, 500 µL of SmartSEC HT isolation buffer was added to each well and the plate was centrifuged again for two minutes at 500 x g. Until further use, samples were stored at -80 °C. As described previously (Hirschberg et al., 2022), SEC fraction 1 is most suitable for mass spectrometry purposes, therefore, this elution fraction was used for the biomarker study. SEC fraction 2, however, was also collected and stored at -80 °C. From this step onwards, a distinction will be made in non-purified CSF (aliquots number two) and CSF-derived EVs (aliquots number one), the latter one being isolated in these steps resulting into two SEC fractions **(Error! Reference source not found.).**

### Protein Concentration

For CSF-derived EV samples, total protein content was determined using the NanoOrange Protein Quantitation Kit (Thermo Scientific) following the manufacturer's specifications for both SEC fraction 1 and SEC fraction 2. A standard curve of serially diluted Bovine Serum Albumin (BSA; Thermo Scientific) was used. EV samples were vacuum dried and re-solved in filtered PBS in a volume 1:3 of the dried-down volume and were diluted 1:50 as a final assay concentration. Signals of all samples, in triplicate, were measured on a Synergy HT microplate reader (Agilent Technologies). Values were extrapolated from the BSA curve, using a linear equation, with r²>0.98.

For non-purified CSF samples, total protein content was determined using UV absorbance at 280nm with a NanoDrop spectrophotometer (NanoDrop 2000, method Protein A280, Thermo Scientific). Here, protein sample quantitation was performed by taking 2 µL of each sample and measured in duplicate. Calculations were performed by the NanoDrop operating software (NanoDrop 2000/2000c version 1.3.1).

### LC-MS/MS sample preparation

For LC-MS/MS sample preparation, the cohort was divided into 18 different sample batches using block randomisation. To gain insight into quality and batch variances of the LC-MS/MS measurements, a reference sample was prepared. For this cause, a pooled sample was prepared, containing different samples of all experimental groups. For CSF-derived EV samples, as there was a limitation in available material, 63 samples of the cohort were selected with the highest protein concentration. Of each of those proteins, 0.75 µg was taken and pooled. For the non-purified CSF pool, 20 µL of all samples out of batch 1 and 2 was used. Pooled samples were stored at -80 °C until sample preparation.

### Protein extraction and S-trap digestions

Each sample was digested in an S-trap micro spin column (Protifi, ref. CO2-micro) according to the manufacturer's instructions. First, 0.75 µg of protein of each CSF-EV derived sample was concentrated in a vacuum dryer (Speedvac SPD1010, Thermo Scientific) and placed in a sonication bath (Branson 3510, Marshall Scientific) for 20 minutes with a final concentration of 5% SDS (Sigma-Aldrich, ref. L3771). For non-purified CSF samples, 10 µL was taken and the same steps were applied.

Proteins were reduced with a final concentration of 10 mM DTT (Sigma, ref. D0632) for 30 minutes at 55 °C with 450 rpm in a Thermomixer C (Eppendorf), followed by an alkylation step with a final concentration of 20 mM iodoacetamide (Sigma, ref. I1149) for at least 30 minutes at room temperature in the dark. The alkylated proteins were acidified by adding phosphoric acid (Biosolve, ref. 161605) to a final concentration of 2.7% and mixed with six volumes of binding buffer (90% methanol, BioSolve, ref. 136841; final concentration of 100 mM TEAB, Sigma, ref. T7408). After vortexing, the solution was loaded onto the S-trap filter and centrifuged at 4,000 x g for 30 seconds. After all sample was loaded, three washing steps with each time 150 µL of the binding buffer were performed followed by a centrifugation step of 4,000 x g for 30 seconds, ending with an additional centrifugation step of 4,000 x g for 1 minute. An overnight digestion with 0.5 µg (CSF-EV derived samples) or 1.0 µg (non-purified CSF samples) Trypsin Platinum (Promega, ref. VA9000) diluted in 20 µL 50 mM TEAB per sample was performed at 37 °C in a humid environment to limit evaporation. Finally, peptides were eluted stepwise with three elution buffers at a volume of 40 µL each, including 50 mM TEAB in water, 0.2% formic acid (Biosolve, ref. 069141A8) in water and 50% acetonitrile (Biosolve, ref. 01204101) / 0.1% formic acid in water. The peptide solutions were pooled, speed-vacuum dried, and subsequently stored at -20 °C. All water used in this protocol was HPLC-graded water (Biosolve, ref. 232141B1).

### LC-MS/MS analysis

All dry CSF-derived EV samples were resuspended in 20 µL of Solvent A (0.1% formic acid in water, Biosolve, ref. 23244102), and all material was used for further steps. Dry non-purified CSF samples were resuspended in 22 µL Solvent A. With 2 µL, peptide concentration was measured using the NanoDrop spectrophometer, and 0.3 µg of peptide was taken to load onto the column.

Before injecting all samples into the mass spectrometer, samples were loaded on Evotips Pure (Evosep, EV2013) according to manufacturer's instructions (Evotip Pure sample loading protocol, downloaded 13.06.2022). In short, Evotips Pure were washed with Solvent B (0.1% formic acid in acetonitrile, Biosolve, ref. 01930602), and conditioned in 1-propanol (Biosolve, ref. 16360602). After equilibrating with Solvent A, the required volume of peptides was loaded onto the tip. Following a centrifugation step of 800 x g for one minute, tips were washed with Solvent A and additional solvent A was added to avoid drying of the tip. Finally, 1 pmol/peptide from the Retention Time standardisation kit PROCAL (JPT, ref. RTK-1-10 pmol) was added to all samples. A peptide mixture from a HeLa cell culture was used as QC sample at the beginning and end of every batch, and a sample with GFP (Protea biosciences, MorganDown, WV) was run every three samples.

The peptide samples were separated on a Evosep One (Evosep), fitted with an ReproSil-Pur C18 Endurance column (15 cm, 1.9 µm, 150 µm, Evosep, EV1113). The pre-programmed Evosep 30 SPD method was used. The column was online with a timsTOF Pro operating in positive ion mode, coupled with a CaptiveSpray ion source (both from Bruker Daltonics GmbH, Bremen). The timsTOF Pro was calibrated according to the manufacturer's guidelines. The temperature of the ion transfer capillary was 180 °C. The Parallel Accumulation-Serial Fragmentation (PASEF) DDA method was used to select precursor ions for fragmentation with 1 TIMS-MS scan and 10 PASEF MS/MS scans. The TIMS-MS survey scan was acquired between 0.70 - 1.45 V.s/cm² and 100 - 1,700 m/z with a ramp time of 100 ms. The 10 PASEF scans contained on average 12 MS/MS scans per PASEF scan with a collision energy of 10 eV. Precursor ions with 1 - 5 charges were selected with the target value set to 20 000 a.u and intensity threshold to 2,500 a.u. Precursors were dynamically excluded for 0.4 s. The timsTOF Pro was controlled by the OtofControl 5.1 software (Bruker Daltonik GmbH). 10 PASEF scans contained on average 12 MS/MS scans per PASEF scan. Raw data was analysed with the DataAnalysis 5.1 software (Bruker Daltonik).

### Peptide identification

A peptide search was performed on a selection of 50 CSF-derived EV samples. The Andromeda search engine from MaxQuant version 2.2.0.0 (Max-Planck Institute, Germany) was used to perform database searches against the database Uniprot Human (Proteome ID: UP000005640, downloaded on 30th January 2020), with a protein FDR of 1%. Methionine oxidation and serine, threonine and tyrosine phosphorylation were set as variable modifications and cysteine carbamidomethylation as a fixed modification. During the search, the trypsin digest was allowed to have two missed cleavage sites. Identifications were transferred between runs with the match-between-runs setting. The MaxQuant .msms output file was used to generate a spectral library with BiblioSpec implemented in the Skyline environment. Here, peptides shared between multiple protein groups were removed and only protein groups with minimally 2 peptides were considered. The raw data of all other 199 CSF-EV derived samples (249 total samples minus the 50 samples already run on MaxQuant) were imported in this spectral library. The same was done for non-purified CSF samples: a selection of 50 samples was run on MaxQuant, with which a spectral library in Skyline was made for the other 198 samples. The use of such spectral library helps avoiding missing values, an issue often encountered in label-free quantitative proteomics.

### Differential analysis

Further data analysis was performed in R version 2022.12.0+353 (Posit). The peptide intensities acquired by Skyline were log2-transformed, filtered for maximum 50% missing values, normalised using the quantile method, and summarised into protein expression values. Package limma (version 3.52.4) from Bioconductor was used for the actual differential analysis. Proteins were considered differentially expressed if the Benjamini-Hochberg-adjusted P-value was lower than 0.05 and the absolute log2 fold change (LogFC) was higher than 1.

### GO ENRICHMENT

DAVID Bioinformatics Resources 2021 (https://david.ncifcrf.qov) was used to perform GO (gene ontology) analysis. The whole proteome background was retrieved from the database Uniprot Human (Proteome ID: UP000005640, downloaded on 30th January 2020).

### Results

### Protein and peptide concentrations

On average, the protein concentration of CSF-derived EVs was 4.57 µg/mL. Of each sample, 0.75 µg was allocated for protein extraction and peptide digestion (i.e. LC-MS/MS sample preparation). The protein concentration of non-purified CSF was about 1,000 times higher, with an average of 4.19 mg/mL. Since 20 µL of these samples was taken for LC-MS/MS sample preparation, on average 41.94 µg of protein was taken. Measurements at peptide level resulted in an average concentration of 0.81 mg/mL, which means that on average 17.79 µg of peptide was retrieved after sample preparation, or 42.42% of the initial protein material. The descriptive statistics can be found in Table 2.

**Table 2. Descriptive statistics of protein and peptide concentration measurements.**

| **Sample type** | **CSF-derived EVs** | **Non-purified CSF** | |
|---|---|---|---|
| | **Protein concentration [µg/mL]** | **Protein concentration [mg/mg]** | **Peptide concentration [mg/mL]** |
| **Minimum** | 0.10 | 0.54 | 0.31 |
| **25% Percentile** | 2.61 | 2.11 | 0.72 |
| **Median** | 3.74 | 2.93 | 0.86 |
| **75% Percentile** | 5.84 | 4.51 | 0.91 |
| **Maximum** | 22.44 | 27.86 | 1.06 |
| | | | |
| **Mean** | 4.571 | 4.194 | 0.8085 |
| **Std. Deviation** | 3.377 | 3.933 | 0.1487 |
| **Std. Error of Mean** | 0.1966 | 0.2309 | 0.008660 |
| | | | |
| **Coefficient of variation** | 73.87% | 93.77% | 18.40% |

Protein concentration of CSF-derived EVs and non-purified CSF did not correlate significantly according to a Spearman test (rho = 0.106, P-value = 0.095). However, as expected, there was a significant correlation between the protein and peptide concentrations of non-purified CSF (rho = 0.241 , P-value = 7.322E-005). This shows the reproducibility of the sample preparation protocol and the amount of material lost. Between the different phenotypes, no significant difference was measured for protein or peptide measurement in both the CSF-derived EV samples and the non-purified CSF **(Error! Reference source not found.).** These statistics were performed both with and without removing outliers (ROUT method, Q = 1%).

### LC-MS/MS

### Identifications

The number of proteins identified and quantified was 350 in non-purified CSF, whereas this number increased to 658 proteins for CSF-derived EVs. Of these identifications, 147 proteins overlapped between both datasets.

### Database comparisons

In this section, al detected proteins in both sample types were compared to databases of known CSF, brain and EV proteins. Two different CSF databases were used, one published by Macron and colleagues (Macron et al., 2020), resulting in 127 proteins overlapping with both our datasets. From the CSF-derived EV proteins, 54.4% were found in this dataset, from non-purified CSF 74.9%. A second CSF database was taken from Guldbrandsen et al. (https://proteomics.uib.no/csf-pr/), with 120 overlapping. From the CSF-derived EV proteins, 41.0% were found in this dataset, from non-purified CSF 72.9%. The brain proteome was used from Protein Atlas comprising of proteins detected in brain, but not in all other tissues (https://www.proteinatlas.org/humanproteome/brain/human+brain), resulting in an overlap of 55 proteins between the three datasets. From the CSF-derived EV proteins, 21.3% were found in this dataset, from non-purified CSF 46.3%. In order to check the number of EV-associated proteins, the dataset DAVID uses to annotate proteins to the GO term extracellular vesicle was used. Here, 103 proteins overlapped between the three datasets. From the CSF-derived EV proteins, 50.3% were found in this dataset, from non-purified CSF 42.0%. From the Vesiclepedia top 100 EV proteins (of which 97 are Swiss-Prot reviewed and included in this comparison), 66.0% was detected in neither datasets, while 34% was detected in the CSF-derived EV samples and only 11.3% was detected in the non-purified CSF samples.

### Differential analysis

All included groups (HC, AD, PD, PD-MCI, PDD, DLB) were compared, resulting in 15 comparisons for each protein, per sample type. For non-purified CSF, 169 statistically significant proteins were detected over all groups in total. Only for AD vs. PD-MCI and PD-MCI vs. PDD, no differential proteins were found. For CSF-derived EVs, 276 statistically significant proteins were detected over all groups. Here, for AD vs. PD-MCI, AD vs. PDD, PD-MCI vs. PDD, and PD vs. PD-MCI, no differential proteins were discovered. Between both sample types, almost no overlap was found in differential proteins .

### GO enrichment

To investigate the characteristics of the CSF-derived EV proteome and non-purified CSF proteome, we employed DAVID using a human proteome reference list. Among the overrepresented GO classes, we found overrepresentation of entries related to ribosomes, extracellular space, melanosome, secretory granule lumen, ficolin-1-rich granule lumen, cytoplasm, extracellular region, focal adhesion, cytosol, and extracellular exosomes (sorted by P-value). For the non-purified CSF, we found entries related to melanosomes, ribosomes, cytosol, blood microparticles, cell surface, endoplasmic reticulum lumen, extracellular matrix, (external side of) plasma membrane, circulating immunoglobulin complex. In regard to the cellular component, almost half of CSF-derived EV proteins were related to the cytoplasm, while for non-purified CSF about half were secreted. For both sample types, enrichment between all detected proteins and differential proteins were comparable.

### Selection of biomarkers

To identify the most promising markers out of these lists of differential proteins, a selection process was carried out based on the comparisons where the protein was significant. The first selection focused on markers for neurodegeneration in general (see Table 3 "neurodegeneration"). Here, the proteins were selected that showed a significant result between comparison HC vs AD, HC vs DLB, HC vs PD, HC vs PD-MCI, and HC vs PDD. Among non-purified CSF differential proteins, two markers were chosen, while 33 met the criteria among CSF-derived EV proteins. For AD markers (see Table "AD"), the selection aimed for markers that could make a distinction between HC vs AD and/or AD vs DLB, while excluding those associated with non-AD diseases. This resulted in 10 potential markers in non-purified CSF and four in CSF-derived EVs. To identify DLB- specific markers (see Table , "DLB"), hypothesizing that this is a different disease from PDD, markers were selected if they differentiated HC vs DLB and AD vs DLB, while not differentiating between non DLB-diseases. This led to the selection of 15 DLB non-purified CSF markers and nine DLB CSF-derived EV markers. For PD (see Table "PD"), the criteria were to differentiate between HC vs PD and PD vs PDD, and not for non-PD diseases. In this case, four non-purified CSF markers were noted and 13 CSF-derived EV markers. For PDD (see Table "PDD"), hypothesizing that this is a different disease from DLB, the focus was on markers that differentiated HC vs PDD and AD vs PDD or DLB vs PDD, while excluding markers associated with non-PD or non-PDD diseases. Five non-purified CSF markers were chosen, and three for CSF-derived EVs. These markers did not appear in the MCI stage of PD. Markers that could potentially measure dementia already in the PD-MCI stage, were selected based on differentiation between both HC vs PD-MCI and HC vs PDD (see Table "PDMCI-PDD"): three identifications for non-purified CSF and five for CSF-derived EVs. Lastly, hypothesizing that DLB and PDD are actually one and the same disease, a selection was made based on markers that show significance for HC vs the PD continuum and HC vs DLB, while excluding proteins that could also differentiate AD (see Table "PDMCI-DLB-PDD").

**Table 3. Selection of most promising protein biomarker candidates (annotated with their gene name) for both non-purified CSF and CSF-derived EVs.**

| | **Non-purified CSF** | **CSF-derived EVs** |
|---|---|---|
| **Neurodegeneration** | IGHV3-48, SYNRG (N = 2) | TAGLN, KRT8, SRI, NUTF2, SELENBP1, RPS18*, IGLC7, TMSB4X*, SF3A1*, HRNRNPK, STMN, NCL*, RPL7*, ACIN1, RPL31*, COL28A1, FABP6*, EIF3D*, PPT1, RPL22*, TRA2B*, SUMO2*, MLF2, ST13P4, MESD, |
| | | CCT5*, S100A6, PSMD2*, HMGB1P1, ACLY*, MYO6*, PSMA7*, RNASE4 (N = 33) |
| **AD** | IGKV3D-15, CA3, SORCS3, MYH9*, TUBB2B, GALNT1, CALML5, SMOC1, SPINT2*, BAMBI* (N = 10) | CFHR2, EDF1, PLA2G4E, ARGLU1 (N = 4) |
| **DLB** | PLXDC2, SHISA5, CPLANE1, PCDH9, C1RL, SERPINB4*, ACTC1, MGP, CD81, IGHV3-30-5, PRKCSH*, SLPI, F2R*, IGHV2-70D, SPOCK1 (N =15) | SAMD9, SIRPA, PREX1*, LDHA, TCOF1, EPHB1, RB1CC1, KRT40, KRT38 (N = 9) |
| **PD** | EPHB6*, KRT8, EPB42, CENPE (N = 4) | RALB, POR, SMPD3, MAPT*, DDX6*, ILK*, OGN", CTSS*, PPP1CC*, FLII, IGKV1-8, SHMT1, RPL35A* (N = 13) |
| **PDD** | SNCA, MASP1*, PLD4, PTPRS, CADM2 (N = 5) | EHD2, ACTBL2, SLIRP (N= 3) |
| **PDMCI-PDD** | HEG1, MOG, PNP* (N = 3) | XIRP2, HP*, IGG1, SORL1*, TF (N = 5) |
| **PDMCI-DLB-PDD** | WFIKKN2, PTPRN2 (N = 2) | TGM5, IGLC1, ALB* (N = 3) |

| | | |
|---|---|---|
| Abbreviations: AD, Alzheimer's disease; PD, Parkinson's disease; PD-MCI, Parkinson's disease with cognitive deficit; PDD, Parkinson's disease dementia; DLB, dementia with Lewy bodies; HC, healthy controls. An asterisk (*) highlights the proteins that showed up in the network analysis as well. Neurodegeneration: differentiate HC vs AD, HC vs DLB, HC vs PD, HC vs PD-MCI, and HC vs PDD. AD: differentiated HC vs AD and/or AD vs DLB, and not non-AD diseases. DLB: differentiated HC vs DLB and AD vs DLB, and not non-DLB diseases. PD: differentiated HC vs PD and PD vs PDD, and not non-PD diseases. PDD: differentiated HC vs PDD and AD vs PDD or DLB vs PDD, and not non-PD or non-PDD diseases. PDMCI-PDD: differentiated HC vs PD-MCI and HC vs PDD. PDMCI-DLB-PDD: differentiated HC vs the PD continuum and HC vs DLB. | | |

### Conclusion

We observed a greater number of differentially expressed proteins in CSF-derived EV samples (N = 276) compared to non-purified CSF (N = 169), with minimal overlap between both datasets. This finding suggests that CSF-derived EV samples may be more suitable for the discovery phase of a biomarker study, due to the removal of more abundant proteins, resulting in a narrower dynamic range. Notably, a larger proportion of the potential biomarkers in CSF-derived EVs, pointed towards neurodegeneration, or in extend to a state of disease in general. For instance, several ribosomal proteins showed altered expression, which in many diseases show up as altered.

Our findings suggest that CSF-derived EVs spread disease-altering proteins, albeit not necessarily the most disease-specific ones. However, it is crucial to emphasise that a comprehensive discussion on this topic can only be conducted after thorough validation of our identified proteins. As more disease-specific markers, we selected a total of 39 markers as the most promising markers identified in non-purified CSF, and 37 markers across the different included diseases in the CSF-derived EV data.

Since of all selected proteins, only nine showed up as known genetic biomarkers, it is necessary to validate all proteins in a larger, independent cohort study. This should be performed on both sample types since further investigations are inquired to determine whether the CSF-derived EV potential biomarkers can also be detected in non-purified CSF using targeted proteomics. This way, a comprehensive understanding of the biomarker's presence and detectability can be obtained. When considering targeted proteomics, non-purified samples may offer a more practical solution as it requires a smaller amount of material compared to EV-enriched samples. Furthermore, it is important to consider other, less-invasive biofluids, such as plasma, or more specific brain-derived EVs from plasma.

### References

Aarsland D, Brønnick K, Ehrt U, De Deyn PP, Tekin S, Emre M, Cummings JL. Neuropsychiatric symptoms in patients with Parkinson's disease and dementia: frequency, profile and associated care giver stress. J Neurol Neurosurg Psychiatry. 2007 Jan;78(1):36-42. doi: 10.1136/jnnp.2005.083113. Epub 2006 Jul 4. PMID: 16820421; PMCID: PMC2117797.
American Psyciatric Association. DSM-IV-TR: Diagnostic and Statistical Manual of Mental Disorders, American Psyciatric Association. 2010.
Devanand DP, Lee S, Huey ED, Goldberg TE. Associations Between Neuropsychiatric Symptoms and Neuropathological Diagnoses of Alzheimer Disease and Related Dementias. JAMA Psychiatry. 2022 Apr 1;79(4):359-367. doi: 10.1001/jamapsychiatry.2021.4363. PMID: 35171235; PMCID: PMC8851371.
Engelborghs S, De Vreese K, Van de Casteele T, Vanderstichele H, Van Everbroeck B, Cras P, Martin JJ, Vanmechelen E, De Deyn PP. Diagnostic performance of a CSF-biomarker panel in autopsy-confirmed dementia. Neurobiol Aging. 2008 Aug;29(8):1143-59. doi: 10.1016/j.neurobiolaging.2007.02.016. Epub 2007 Apr 10. PMID: 17428581.
Engelborghs S, Marescau B, De Deyn PP. Amino acids and biogenic amines in cerebrospinal fluid of patients with Parkinson's disease. Neurochem Res. 2003 Aug;28(8):1145-50. doi: 10.1023/a:1024255208563. PMID: 12834252.
Hirschberg, Y., Boonen, K., Schildermans, K., van Dam, A., Pintelon, I., Vandendriessche, C., Velimirovic, M., Jacobs, A., Vandenbroucke, R. E., Nelissen, I., Vermeiren, Y., & Mertens, I. (2022). Characterising extracellular vesicles from individual low volume cerebrospinal fluid samples, isolated by SmartSEC. Journal of Extracellular Biology, 1, e55. https://doi.org/10.1002/jex2.55
Iketani R, Kawasaki Y, Yamada H. Comparative Utility of Atypical Antipsychotics for the Treatment of Psychosis in Parkinson's Disease: A Systematic Review and Bayesian Network Meta-analysis. Biol Pharm Bull. 2017;40(11): 1976-1982. doi: 10.1248/bpb.b17-00602. PMID: 29093347.
Kawashima S; RCIP-Nagoya Study Group; Matsukawa N. Memantine for the patients with mild cognitive impairment in Parkinson's disease: a pharmacological fMRI study. BMC Neurol. 2022 May 13;22(1):175. doi: 10.1186/s12883-022-02699-x. PMID: 35562711; PMCID: PMC9103297.
Litvan I, Goldman JG, Tröster Al, Schmand BA, Weintraub D, Petersen RC, Mollenhauer B, Adler CH, Marder K, Williams-Gray CH, Aarsland D, Kulisevsky J, Rodriguez-Oroz MC, Burn DJ, Barker RA, Emre M. Diagnostic criteria for mild cognitive impairment in Parkinson's disease: Movement Disorder Society Task Force guidelines. Mov Disord. 2012 Mar;27(3):349-56. doi: 10.1002/mds.24893. Epub 2012 Jan 24. PMID: 22275317; PMCID: PMC3641655.
Macron C, Lavigne R, Núñez Galindo A, Affolter M, Pineau C, Dayon L. Exploration of human cerebrospinal fluid: A large proteome dataset revealed by trapped ion mobility time-of-flight mass spectrometry. Data Brief. 2020 May 16;31:105704. doi: 10.1016/j.dib.2020.105704. PMID: 32478154; PMCID: PMC7251648.
McKhann GM, Knopman DS, Chertkow H, Hyman BT, Jack CR Jr, Kawas CH, Klunk WE, Koroshetz WJ, Manly JJ, Mayeux R, Mohs RC, Morris JC, Rossor MN, Scheltens P, Carrillo MC, Thies B, Weintraub S, Phelps CH. The diagnosis of dementia due to Alzheimer's disease: recommendations from the National Institute on Aging-Alzheimer's Association workgroups on diagnostic guidelines for Alzheimer's disease. Alzheimers Dement. 2011 May;7(3):263-9. doi: 10.1016/j.jalz.2011.03.005. Epub 2011 Apr 21. PMID: 21514250; PMCID: PMC3312024.
McKeith IG, Boeve BF, Dickson DW, Halliday G, Taylor JP, Weintraub D, Aarsland D, Galvin J, Attems J, Ballard CG, Bayston A, Beach TG, Blanc F, Bohnen N, Bonanni L, Bras J, Brundin P, Burn D, Chen-Plotkin A, Duda JE, El-Agnaf O, Feldman H, Ferman TJ, Ffytche D, Fujishiro H, Galasko D, Goldman JG, Gomperts SN, Graff-Radford NR, Honig LS, Iranzo A, Kantarci K, Kaufer D, Kukull W, Lee VMY, Leverenz JB, Lewis S, Lippa C, Lunde A, Masellis M, Masliah E, McLean P, Mollenhauer B, Montine TJ, Moreno E, Mori E, Murray M, O'Brien JT, Orimo S, Postuma RB, Ramaswamy S, Ross OA, Salmon DP, Singleton A, Taylor A, Thomas A, Tiraboschi P, Toledo JB, Trojanowski JQ, Tsuang D, Walker Z, Yamada M, Kosaka K. Diagnosis and management of dementia with Lewy bodies: Fourth consensus report of the DLB Consortium. Neurology. 2017 Jul 4;89(1):88-100. doi: 10.1212/WNL.0000000000004058. Epub 2017 Jun 7. PMID: 28592453; PMCID: PMC5496518.
Müller ML, Bohnen NI. Cholinergic dysfunction in Parkinson's disease. Curr Neurol Neurosci Rep. 2013 Sep;13(9):377. doi: 10.1007/s11910-013-0377-9. PMID: 23943367; PMCID: PMC3991467.
Pasquini J, Brooks DJ, Pavese N. The Cholinergic Brain in Parkinson's Disease. Mov Disord Clin Pract. 2021 Aug 23;8(7):1012-1026. doi: 10.1002/mdc3.13319. PMID: 34631936; PMCID: PMC8485627.
Smirnov DS, Galasko D, Edland SD, Filoteo JV, Hansen LA, Salmon DP. Cognitive decline profiles differ in Parkinson disease dementia and dementia with Lewy bodies. Neurology. 2020 May 19;94(20):e2076-e2087. doi: 10.1212/WNL.0000000000009434. Epub 2020 Apr 24. PMID: 32332125; PMCID: PMC7526670.
Vermeiren Y, Le Bastard N, Van Hemelrijck A, Drinkenburg WH, Engelborghs S, De Deyn PP. Behavioral correlates of cerebrospinal fluid amino acid and biogenic amine neurotransmitter alterations in dementia. Alzheimers Dement. 2013 Sep;9(5):488-98. doi: 10.1016/j.jalz.2012.06.010. Epub 2012 Nov 14. PMID: 23159046.

## Claims

1. A method of differentiating cognitive impairment patients with Alzheimers's Disease (AD), Parkinson's Disease (PD), and Lewy Body Dementia (DLB) from one another, said method comprising determining within a CSF-derived EV protein sample or within a non-purified CSF protein sample of said patient the presence of;
- at least one protein from the group consisting of IGKV3D-15, CA3, SORCS3, MYH9, TUBB2B, GALNT1, CALML5, SMOC1, SPINT2, BAMBI, CFHR2, EDF1, PLA2G4E, and ARGLU1;
- at least one protein from the group consisting of PLXDC2, SHISA5, CPLANE1, PCDH9, C1RL, SERPINB4, ACTC1, MGP, CD81, IGHV3-30-5, PRKCSH, SLPI, F2R, IGHV2-70D, SPOCK1, SAMD9, SIRPA, PREX1, LDHA, TCOF1, EPHB1, RB1CC1, KRT40, and KRT38; and
- at least one protein from the group consisting of EPHB6, KRT8, EPB42, CENPE, RALB, POR, SMPD3, MAPT, DDX6, ILK, OGN, CTSS, PPP1CC, FLII, IGKV1-8, SHMT1, and RPL35A.

2. The method according to claim 1, further comprising determining within a CSF-derived EV protein sample or within a non-purified CSF protein sample of said patient the presence of;
- at least one protein from the group consisting of SNCA, MASP1, PLD4, PTPRS, CADM2, EHD2, ACTBL2, and SLIRP;
- at least one protein from the group consisting of HEG1, MOG, PNP, XIRP2, HP, IGG1, SORL1, and TF; and
- at least one protein from the group consisting of WFIKKN2, PTPRN2, TGM5, IGLC1, and ALB; to identify Parkinson's Disease Mild Cognitive Impairment (PDMCI) and Parkinson's Disease Dementia (PDD) in cognitive impairment patients.

3. The method of claim 1, wherein the method is only based on the use of non-purified CSF protein samples, said method comprises determining within a non-purified CSF protein sample of said patient the presence of;
- at least one protein from the group consisting of IGKV3D-15, CA3, SORCS3, MYH9, TUBB2B, GALNT1, CALML5, SMOC1, SPINT2 and BAMBI;
- at least one protein from the group consisting of PLXDC2, SHISA5, CPLANE1, PCDH9, C1RL, SERPINB4, ACTC1, MGP, CD81, IGHV3-30-5, PRKCSH, SLPI, F2R, IGHV2-70D and SPOCK1; and
- at least one protein from the group consisting of EPHB6, KRT8, EPB42 and CENPE.

4. The method of claim 2, wherein the method is only based on non-purified CSF protein samples, said method comprises determining within a non-purified CSF protein sample of said patient the presence of;
- at least one protein from the group consisting of SNCA, MASP1, PLD4, PTPRS and CADM2;
- at least one protein from the group consisting of HEG1, MOG and PNP; and
- at least one protein from the group consisting of WFIKKN2 and PTPRN2.

5. The method of claim 1, wherein the method is only based on the use of CSF-derived EV protein samples, said method comprises determining within a CSF-derived EV protein sample of said patient the presence of;
- at least one protein from the group consisting of CFHR2, EDF1, PLA2G4E, and ARGLU1;
- at least one protein from the group consisting of SAMD9, SIRPA, PREX1, LDHA, TCOF1, EPHB1, RB1CC1, KRT40, and KRT38; and
- at least one protein from the group consisting of RALB, POR, SMPD3, MAPT, DDX6, ILK, OGN, CTSS, PPP1CC, FLII, IGKV1-8, SHMT1, and RPL35A.

6. The method of claim 2, wherein the method is only based on CSF-derived EV protein samples, said method comprises determining within a CSF-derived EV protein sample of said patient the presence of;
- at least one protein from the group consisting of EHD2, ACTBL2, and SLIRP;
- at least one protein from the group consisting of XIRP2, HP, IGG1, SORL1, and TF; and
- at least one protein from the group consisting of TGM5, IGLC1, and ALB.

7. The method according to any one of the preceding claims, wherein the identification of cognitive impairment patients with Alzheimers's Disease (AD) is based on determining within a CSF-derived EV protein sample or within a non-purified CSF protein sample of said patient the presence of at least one protein from the group consisting of IGKV3D-15, CA3, SORCS3, MYH9, TUBB2B, GALNT1, CALML5, SMOC1, SPINT2, BAMBI, CFHR2, EDF1, PLA2G4E, and ARGLU1. In particular the presence of at least one protein selected from the group consisting of IGKV3D-15, CA3, SORCS3, MYH9, TUBB2B, GALNT1, CALML5, SMOC1, SPINT2, and BAMBI in a non-purified CSF protein sample of said patient and/or at least one protein selected from the group consisting of CFHR2, EDF1, PLA2G4E, and ARGLU1 in a CSF-derived EV protein sample of said patient.

8. The method according to any one of the preceding claims wherein the identification of cognitive impairment patients with Lewy Body Dementia (DLB) is based on determining within a CSF-derived EV protein sample or within a non-purified CSF protein sample of said patient the presence of at least one protein from the group consisting of PLXDC2, SHISA5, CPLANE1, PCDH9, C1RL, SERPINB4, ACTC1, MGP, CD81, IGHV3-30-5, PRKCSH, SLPI, F2R, IGHV2-70D, SPOCK1, SAMD9, SIRPA, PREX1, LDHA, TCOF1, EPHB1, RB1CC1, KRT40, and KRT38. In particular the presence of at least one protein selected from the group consisting of PLXDC2, SHISA5, CPLANE1, PCDH9, C1RL, SERPINB4, ACTC1, MGP, CD81, IGHV3-30-5, PRKCSH, SLPI, F2R, IGHV2-70D, and SPOCK1 in a non-purified CSF protein sample of said patient and/or at least one protein selected from the group consisting of SAMD9, SIRPA, PREX1, LDHA, TCOF1, EPHB1, RB1CC1, KRT40, and KRT38 in a CSF-derived EV protein sample of said patient.

9. The method according to any one of the preceding claims wherein the identification of cognitive impairment patients with Parkinson's Disease (PD) is based on determining within a CSF-derived EV protein sample or within a non-purified CSF protein sample of said patient the presence of at least one protein from the group consisting of EPHB6, KRT8, EPB42, CENPE, RALB, POR, SMPD3, MAPT, DDX6, ILK, OGN, CTSS, PPP1CC, FLII, IGKV1-8, SHMT1, and RPL35A. In particular the presence of at least one protein selected from the group consisting of EPHB6, KRT8, EPB42, and CENPE in a non-purified CSF protein sample of said patient and/or at least one protein selected from the group consisting of RALB, POR, SMPD3, MAPT, DDX6, ILK, OGN, CTSS, PPP1CC, FLII, IGKV1-8, SHMT1, and RPL35A in a CSF-derived EV protein sample of said patient.

10. The method according to any one of the preceding claims wherein the identification of cognitive impairment patients with Parkinson's Disease Dementia (PDD) is based on determining within a CSF-derived EV protein sample or within a non-purified CSF protein sample of said patient the presence of at least one protein from the group consisting of SNCA, MASP1, PLD4, PTPRS, CADM2, EHD2, ACTBL2, and SLIRP. In particular the presence of at least one protein selected from the group consisting of SNCA, MASP1, PLD4, PTPRS, and CADM2 in a non-purified CSF protein sample of said patient and/or at least one protein selected from the group consisting of EHD2, ACTBL2, and SLIRP in a CSF-derived EV protein sample of said patient.

11. Use of the method according to any one of the preceding claims to differentiate HC vs PDMCI and HC vs PDD, said differentiation comprising determining within a CSF-derived EV protein sample or within a non-purified CSF protein sample of said patient the presence of at least one protein from the group consisting of HEG1, MOG, PNP, XIRP2, HP, IGG1, SORL1, and TF. In particular the presence of at least one protein selected from the group consisting of HEG1, MOG, and PNP in a non-purified CSF protein sample of said patient and/or at least one protein selected from the group consisting ofXIRP2, HP, IGG1, SORL1, and TF in a CSF-derived EV protein sample of said patient.

12. Use of the method according to any one of the preceding claims to differentiate HC vs PD continuum and HC vs DLB (and eventually also AD vs DLB and/or AD vs PD continuum), said differentiation comprising determining within a CSF-derived EV protein sample or within a non-purified CSF protein sample of said patient the presence of at least one protein from the group consisting of WFIKKN2, PTPRN2, TGM5, IGLC1, and ALB. In particular the presence of at least one protein selected from the group consisting of WFIKKN2, and PTPRN2 in a non-purified CSF protein sample of said patient and/or at least one protein selected from the group consisting of TGM5, IGLC1, and ALB in a CSF-derived EV protein sample of said patient.

13. The method or use according to any one of the preceding claims, wherein whenever reference is made to 'determining at least one protein the group consisting of', this includes the determining at least 2, 3, 4, 5, 6, 7, 8, 9, 10, 11, 12, 13, 14, 15, 16, 17, 18, 19, 20, 21, 22, 23, 24 or the total number of proteins present within said group.
